# FASCICULE DE BREVET EUROPEEN

(11) **EP 0 789 074 B1**
(45) Date de publication et mention de la délivrance du brevet: **17.12.2003**
(21) Numéro de dépôt: 97400037.4
(22) Date de dépôt: 08.01.1997
(51) Int. Cl.: C12N 5/06, A61L 27/00

(54) **Equivalent de peau comprenant des cellules de Langerhans**
Hautäquivalent, das Langerhans-Zellen enthält
Skin equivalent containing Langerhans cells

(30) Priorité: 23.01.1996 FR 9600743
(43) Date de publication de la demande: 13.08.1997
(73) Titulaire: L'OREAL, 75008 Paris (FR)
(72) Inventeur: Schmidt, Rainer, 75018 Paris (FR); Schmitt, Daniel, 38540 Heyrieux (FR); Regnier, Marcelle, 75018 Paris (FR); Staquet, Marie-Jeanne, 69150 Decines (FR)
(74) Mandataire: Tezier Herman, Béatrice

(56) Documents cités:
- EP-A- 0 470 681
- WO-A-90/02796

## Description

La présente invention concerne un nouvel équivalent de peau, son procédé d'obtention, l'équivalent d'épiderme compris dans cet équivalent de peau et son procédé de préparation.

On cherche à mettre au point, depuis plusieurs années, des modèles de peau reconstruite qui permettent d'effectuer les études nécessaires à la meilleure compréhension du rôle de la peau tant dans le domaine mécanique que dans le domaine physiologique.

Ainsi, des modèles plus ou moins proches de la peau humaine ont pu être mis au point. On peut citer par exemple les modèles décrits dans les brevets ou dans les demandes de brevets EP 285471, EP 285474, EP 418035, WO-A-90 02796, WO-A-9116010, EP 197090, EP 20753, FR 2665175, FR 2689904.
De manière très générale, les modèles de peau reconstruite décrits dans ces documents sont constitués de kératinocytes humains déposés sur un support, souvent un équivalent de derme, et cultivés dans des conditions telles qu'ils entrent dans un programme de différenciation aboutissant à la formation d'un équivalent d'épiderme.
Cependant, l'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.
Les cellules de Langerhans sont impliquées dans les défenses immunitaires de la peau. On sait depuis longtemps que celles-ci occupent une place essentielle dans les défenses immunitaires de l'hôte, en particulier comme première barrière face aux agressions extérieures.

Les cellules de Langerhans sont des cellules dérivées de la moelle osseuse qui peuvent être caractérisées par la présence de granules de Birbeck et l'expression du marqueur antigénique CD1a (cellules CD1a positives) (Rowden et coll., Nature 268 : 247-248, 1977).
Elles jouent un rôle décisif dans l'initiation des réponses immunitaires dirigées contre les antigènes introduits dans la peau ou nouvellement générés par celle-ci.
Mises en contact avec un allergène, les cellules de Langerhans migrent vers les ganglions où elles déclenchent les réactions spécifiques des cellules-T. A ce titre, elles sont donc assimilées aux cellules de présentation des antigènes, essentielles au bon fonctionnement des lymphocytes T.

Ainsi, la principale fonction des cellules de Langerhans est de fournir un signal sensitif dans la réponse immunitaire de la peau induite contre une grande variété d'antigènes incluant les allergènes de contact, les antigènes tumoraux et les microorganismes.
On peut donc en conclure que ces cellules interviennent probablement dans un grand nombre de pathologies de la peau.

Le document WO-A-90 02796 suggère d'ajouter, dans un système tridimensionnel de culture de peau, à des cultures de kératinocytes et de mélanocytes, des cellules de Langerhans isolées à partir de prélèvements de peau fraîche. Ce même document précise que la croissance en culture de telles cellules est difficile. Il s'avère en fait que les cellules de Langerhans purifiées à partir de prélèvement de peau sont des cellules issues de précurseurs qui ont progressé dans leur cycle de différenciation jusqu'à devenir des cellules CD1a positives et que ces cellules isolées ne progressent plus dans leur cycle de différenciation.
La culture *in vitro* de telles cellules se résume à un maintien en survie du fait de l'incapacité pour ces cellules de se multiplier. En fait ces cellules s'arrêtent et meurent sans avoir rempli leur rôle. Il en est de même lorsque ces cellules sont introduites dans un modèle de peau reconstruite. Ainsi, même s'il est suggéré d'introduire des cellules de Langerhans dans ce document, la peau reconstruite obtenue ne pourrait être satisfaisante, dans la mesure où les cellules de Langerhans ne survivent pas.

Les mélanocytes sont localisés dans la couche basale de l'épiderme. Ils sont le siège de la mélanogénèse et du fait de leur contact étroit avec les kératinocytes ils transfèrent à ceux-ci la mélanine néosynthétisée sous la forme de mélanosomes, donnant ainsi à la peau sa coloration. Le type et la quantité de mélanine contenue dans les mélanosomes détermine la coloration de la peau. De plus, la mélanine constitue principalement un écran efficace pour la protection contre les rayonnements solaires en particulier les rayonnements ultraviolets. Un modèle de peau reconstruite ayant incorporé des mélanocytes est décrit dans la demande de brevet WO-A-9351165.

On comprend donc que les modèles de peau reconstruite décrits dans l'art antérieur ne permettent que des études incomplètes du rôle et/ou des réactions de la peau. En effet, l'absence de cellules de Langerhans, cellules essentielles pour les défenses immunitaires de la peau, dans ces modèles rend ceux-ci inutilisables pour des études immunologiques *in vitro*. Par ailleurs, on peut supposer que les études pharmacologiques et/ou toxicologiques réalisées avec ces modèles n'ont pu refléter qu'une partie de la réalité des interactions du fait de l'absence de ce composant essentiel de la peau que constituent les cellules de Langerhans.

Indépendamment des phénomènes naturels qui conduisent aux symptômes liés aux peaux dites sensibles et/ou allergiques, phénomènes qu'un tel modèle pourra permettre de mieux cerner, l'industrie en général, et celle des cosmétiques en particulier, introduit de plus en plus souvent des composés nouveaux dans ses compositions. Un des problèmes majeurs auquel elle est confrontée est alors l'évaluation des effets néfastes que ces composés pourraient induire au contact de la peau en particulier en terme de sensibilisation de contact.

Pour des raisons d'éthique, il est évident que cette évaluation ne peut être pratiquée ni sur l'homme, ni sur les animaux.

On comprend alors l'intérêt d'un modèle *in vitro* qui puisse permettre, par l'appréhension des mécanismes de défense de la peau, une meilleure compréhension des symptômes liés aux peaux dites sensibles et/ou allergiques et une évaluation des effets néfastes des composés potentiellement utilisables dans l'industrie.

La demanderesse, qui depuis de longues années s'intéresse au domaine des peaux reconstruites, a maintenant découvert qu'il est possible d'incorporer au moins des précurseurs de cellules de Langerhans, préalablement induits ou non, dans un modèle de peau reconstruite.

La présente invention a donc pour objet un modèle de peau reconstruite, caractérisé par le fait qu'il comprend un équivalent d'épiderme sur un support, ledit équivalent d'épiderme comprenant au moins des kératinocytes et au moins des précurseurs de cellules de Langerhans induits ou non.

L'expression "précurseurs de cellules de Langerhans induits " couvre dans ce texte toute cellule, normale ou pathologique, qui a subi, préalablement à sa mise en co-culture, au moins un traitement destiné a lui conférer les caractères d'une cellule de Langerhans, c'est à dire une cellule CD1a positive, sans préjuger de l'acquisition ou non par cette cellule du caractère CD1a positif.

Dans la peau normale, les cellules de Langerhans sont localisées dans la partie suprabasale de l'épiderme.
Préférentiellement, le modèle de peau reconstruite selon l'invention présente au moins des précurseurs de cellules de Langerhans induits ou non localisés dans la partie suprabasale de l'équivalent d'épiderme.

Il est bien entendu que l'équivalent de peau de l'invention peut comprendre tout autre type cellulaire qui pourrait y être incorporé.

La demanderesse a réussi à établir un procédé de préparation d'un équivalent de peau comprenant un équivalent d'épiderme sur un support, ledit équivalent d'épiderme comprenant au moins des kératinocytes et au moins des précurseurs de cellules de Langerhans induits ou non.

L'invention a également pour objet un procédé pour la préparation d'un équivalent de peau tel que décrit ci-dessus, caractérisé par le fait que l'on co-cultive sur le support, des kératinocytes et au moins des précurseurs de cellules de Langerhans induits ou non.

Pour faciliter la compréhension, l'emploi du terme "co-culture" (ou des termes apparentés) doit s'entendre comme se rapportant aux cultures cellulaires effectuées sur le support permettant l'élaboration de la peau reconstruite, sans que cela sous-entende obligatoirement la présence de plus d'une espèce cellulaire. Les termes "culture" et "cultive" s'entendent alors comme se rapportant aux cultures cellulaires effectuées de manière classique par exemple dans des boites de cultures cellulaires traditionnelles.

Le support utilisé selon l'invention peut être l'un quelconque de ceux décrits dans l'art antérieur. A titre d'exemple, on peut citer comme support les lattices mixtes collagène/fibroblastes, le derme préalablement désépidermisé, les membranes artificielles comme par exemple les filtres de marque Millipores, les substituts sous-cutanés à base de collagène, le plastique ou tout autre support compatible avec la viabilité cellulaire.

Préférentiellement, le support est constitué par un derme préalablement désépidermisé.

Quelque soit le support choisi, le protocole pour sa mise en oeuvre utilisé selon l'invention peut-être l'un quelconque des protocoles décrits dans l'art antérieur.

Préférentiellement, selon l'invention lorsque le support est constitué par un derme préalablement désépidermisé on met en oeuvre le protocole décrit dans Prunieras et collaborateurs, Ann. Chir. Plast., 1979, 24, n°4, 357-362.

Selon l'invention, pour obtenir l'équivalent d'épiderme comprenant au moins des kératinocytes et au moins des précurseurs de cellules de Langerhans induits ou non, on co-cultive ensemble sur le support au moins des kératinocytes et au moins des précurseurs de cellules de Langerhans induits ou non.

Les kératinocytes utilisés selon l'invention peuvent être préparés selon toute méthode connue de l'art antérieur. On peut citer à titre d'exemple la culture à partir d'épiderme dissocié provenant de prélèvement de peau normale ou pathologique ou la culture de kératinocytes issus de la gaine de follicules pileux normaux ou pathologiques.

Préférentiellement, selon l'invention les kératinocytes utilisés sont préparés à partir d'épiderme dissocié provenant de prélèvement de peau normale ou pathologique, selon la méthode décrite dans Régnier et collaborateurs, Frontier of Matrix Biology, Vol.9, 4-35 (Karger, Basel 1981).

Les précurseurs de cellules de Langerhans peuvent être toute cellule souche apte à se différencier sous l'effet d'une induction en cellules de Langerhans, c'est à dire apte à se différencier en cellules CD1a positives. Avantageusement, ces précurseurs peuvent être des cellules hématopoïétiques CD34⁺ (Caux et collaborateurs, Nature, Vol. 360, Nov. 1992, 258).

Les précurseurs de cellules de Langerhans peuvent être purifiés à partir des tissus dans lesquels ils se trouvent naturellement, parmi lesquels on peut citer la moelle osseuse, le sang périphérique, le sang de cordon ombilical.

Préférentiellement, on utilise selon le procédé de l'invention des précurseurs de cellules de Langerhans préparés à partir de sang périphérique ou de sang de cordon ombilical et encore plus préférentiellement des précurseurs préparés à partir de sang de cordon ombilical.

Toute méthode de purification peut être utilisée à cette fin. On peut citer, par exemple, celle décrite dans Caux et collaborateurs, Nature, Vol. 360, Nov. 1992, 258.

Selon l'invention, l'induction de la différenciation des précurseurs de cellules de Langerhans peut être réalisée avant ou après leur mise en co-culture.

Cette induction peut bien évidement être réalisée par toute méthode connue. Parmi ces méthodes on peut citer, par exemple, la différenciation induite par les cytokines telles que le facteur de stimulation de colonies (Granulocyte/Macrophage - Colony Stimulating Factor ou GM-CSF), le facteur de nécrose tumorale (Tumor Necrosis Factor ou TNF-α), le facteur de cellules souches (Stem Cell Factor ou SCF), l'interleukine 3 ou encore l'interleukine 4 ou un mélange de celles-ci.

Mais la demanderesse a découvert que de manière tout à fait surprenante la différenciation des précurseurs de cellules de Langerhans peut être spontanément induite par la présence de kératinocytes ou encore par une culture de ces précurseurs dans un milieu dans lequel ont été préalablement cultivés des kératinocytes.

L'induction en présence de kératinocytes peut donc être réalisée soit par une culture simultanée de précurseurs de cellules de Langerhans et de kératinocytes, l'induction intervenant dans la culture, soit par co-culture des précurseurs de cellules de Langerhans et des kératinocytes sur un support, par exemple un derme désépidermisé, l'induction intervenant au sein de la co-culture.

Préférentiellement selon l'invention, l'induction de la différenciation des précurseurs de cellules de Langerhans est réalisée par la présence de kératinocytes et encore plus préférentiellement par co-culture des précurseurs de cellules de Langerhans et des kératinocytes sur un support.

Avantageusement, l'induction de la différenciation des précurseurs de cellules de Langerhans peut s'effectuer par la combinaison d'au moins deux de ces méthodes comme par exemple la culture en présence de kératinocytes et en présence d'au moins une cytokine ou encore la culture en présence d'un mélange de cytokines, comme par exemple la combinaison du GM-CSF et du TNF-α.

La concentration en cytokines utilisée pour l'induction est bien évidemment fonction de la nature de la cytokine utilisée.
Les cytokines sont en générale présentes à des concentrations comprises entre 1 ng/ml et 400 ng/ml de préférence entre 2,5 ng/ml et 300 ng/ml.
Ainsi, par exemple pour le GM-CSF la concentration peut être comprise entre 100 ng/ml et 400 ng/ml et de préférence entre 200 ng/ml et 300 ng/ml. Pour le TNF-α la concentration peut être comprise entre 1 ng/ml et 7,5 ng/ml et de préférence entre 2,5 ng/ml et 5 ng/ml.

Dans la mesure où un mélange de cytokines est utilisé, la proportion d'une cytokine par rapport à l'autre varie en fonction de la nature des cytokines utilisées. Par exemple dans le cas du mélange GM-CSF/TNF-α, celle-ci est comprise entre les rapports pondéraux 400/1 et 13/1 et de préférence entre 120/1 et 40/1.

Il est possible pour la mise en co-culture d'enrichir la préparation de précurseurs induits en cellules CD1a positives. Pour cela on isole dans les cultures de précurseurs induits les cellules CD1a positives.

Selon l'invention, le rapport entre le nombre de kératinocytes et les précurseurs de cellules de Langerhans induits ou non, co-cultivés sur le support, est compris entre les rapport 95/5 et 25/75 en pourcentage du nombre de cellules total dans la co-culture et préférentiellement entre 75/25 et 35/65 et encore plus préférentiellement ce rapport est de 50/50.

Selon le procédé de l'invention, les précurseurs des cellules de Langerhans induits ou non sont mis en co-culture à tout stade de l'élaboration de l'équivalent d'épiderme.

Avantageusement, le procédé selon l'invention comporte une étape dans laquelle les précurseurs des cellules de Langerhans induits ou non sont mis en co-culture avec les kératinocytes en un temps qui permet leur localisation suprabasale dans l'équivalent d'épiderme.

Préférentiellement, les précurseurs des cellules de Langerhans induits ou non sont mis en co-culture à un temps choisi entre le moment où la couche suprabasale de l'équivalent d'épiderme commence à se former et le moment où les kératinocytes différenciés commencent à former la première couche du stratum corneum.

Encore plus préférentiellement, les précurseurs de cellules de Langerhans induits ou non sont mis en co-culture à un temps choisi entre le moment où la couche suprabasale de l'équivalent d'épiderme commence à se former et le deuxième jour d'incubation après exposition à l'interface air/liquide (voir ci-dessous).

Le milieu nutritif utilisé pour le procédé selon l'invention peut être tout milieu nutritif connu pour sa capacité à permettre la prolifération et la différenciation des kératinocytes. On peut citer à titre d'exemple le milieu Dulbecco modifié Eagle, ou un milieu défini à teneur en calcium variable, tel le milieu décrit par Boyce S.T. et Ham R.G., (J. Tissue Cult. Meth., 1985, 9, 83-93).
Avantageusement, selon l'invention il est possible d'utiliser un mélange de plusieurs milieux nutritifs comme par exemple le mélange milieu Dulbecco modifié Eagle/milieu HAM F12 ou milieu de Rheinwald et Green, (Cell, 1975, 6, 331-334).

Selon le protocole de préparation de l'équivalent de peau, la co-culture est d'abord maintenue immergée dans un milieu nutritif pendant un temps d'incubation de 3 à 8 jours. Ce milieu nutritif peut être par exemple le milieu décrit par Rheinwald et Green, milieu qui permet la prolifération des kératinocytes.

Au bout de ce temps d'incubation, la co-culture est portée à l'interface air/liquide, par exemple par dépôt sur une grille métallique. Le liquide est alors préférentiellement constitué du même milieu nutritif que le précédent, à la différence prêt que seuls 3 des facteurs de croissances initialement contenus dans le milieu de Rheinwald et Green sont conservés aux mêmes concentrations, à savoir le facteur de croissance épidermique (EGF), l'insuline et l'hydrocortisone.

L'incubation se poursuit ensuite jusqu'à obtention d'un équivalent de peau présentant les caractéristiques d'une peau, à savoir un équivalent de derme supportant un équivalent d'épiderme présentant les couches cellulaires classiques à savoir les couches basale, suprabasale, granuleuse et cornée.

Ainsi, l'incubation se poursuit pendant une durée comprise entre 5 et 30 jours.

Le modèle de peau reconstruite ainsi réalisé est constitué de deux entités, le support et l'équivalent d'épiderme, qu'il est possible de séparer physiquement l'un de l'autre.
L'équivalent d'épiderme peut alors être utilisé séparément du support.

L'invention concerne donc également un équivalent d'épiderme, caractérisé par le fait qu'il comprend au moins des kératinocytes et au moins des précurseurs de cellules de Langerhans induits ou non et son procédé de préparation, tel que décrit ci-dessus.

Préférentiellement, l'équivalent d'épiderme selon l'invention présente au moins des précurseurs de cellules de Langerhans induits ou non localisés dans sa partie suprabasale.

Bien entendu, l'équivalent de peau qui présente la meilleure similitude avec la peau normale est l'équivalent de peau qui contient les trois types cellulaires essentiels présents dans la peau normale.

Ainsi, avantageusement, le modèle de peau reconstruite selon l'invention comprend en outre des mélanocytes.

L'invention concerne avantageusement un équivalent d'épiderme contenant des kératinocytes, au moins des précurseurs de cellules de Langerhans induits ou non et des mélanocytes.

Les mélanocytes utilisés selon l'invention peuvent être purifiées à partir de tout organe en contenant comme par exemple la peau normale ou le follicule de cheveu.

Préférentiellement, on utilise des mélanocytes purifiés à partir de peau normale.

Toute méthode de purification connue de l'art antérieur peut être utilisée pour préparer ces mélanocytes. On peut citer par exemple la méthode décrite dans Olsson et collaborateurs, Acta Derm. Venereol., 1994, 74, 226-268.

Pour obtenir un équivalent de peau contenant les trois types cellulaires présents dans la peau normale, il est nécessaire de co-cultiver des kératinocytes, des précurseurs de cellules de Langerhans induits ou non et des mélanocytes sur un support.

Ainsi, selon un autre mode particulier du procédé de l'invention, celui-ci se caractérise par le fait que l'on co-cultive sur un support, des kératinocytes, au moins des précurseurs de cellules de Langerhans induits ou non et des mélanocytes.

Dans les cas d'un équivalent de peau comprenant les trois types cellulaires, le procédé mis en oeuvre pour sa réalisation ne diffère en rien de celui exposé précédemment pour la réalisation d'un équivalent de peau comprenant deux types cellulaires.
Par exemple le rapport entre le mélange (kératinocytes+mélanocytes) et les cellules de Langerhans et/ou les précurseurs co-cultivés sur le support est compris entre les rapport 95/5 et 25/75 en pourcentage du nombre total de cellules dans la co-culture et préférentiellement entre 75/25 et 35/65 et encore plus préférentiellement ce rapport est de 50/50.

Le rapport particulier entre kératinocytes et mélanocytes peut être celui décrit dans l'art antérieur (WO-A-9351165).

Les exemples ci-dessous illustrent l'invention sans la limiter aucunement.

### Exemple de préparation d'un équivalent d'épiderme contenant des kératinocytes, des cellules de Langerhans et des mélanocytes :

On constitue un mélange de kératinocytes humains normaux préalablement isolés selon la méthode décrite dans Régnier et collaborateurs, (Frontier of Matrix Biology, Vol.9, 4-35, Karger, Basel 1981) et de mélanocytes humains préalablement isolés selon la méthode décrite dans Olsson et coll., (Acta Derm. Venereol., 1994, 74, 226-268), dans une proportion de 10 pour 1. Ce mélange est déposé sur un derme désépidermisé à raison de 5x10⁵ cellules par cm², selon la méthode décrite dans Prunieras et collaborateurs, Ann. Chir. Plast., 1979, 24, n°4, 357-362. La culture s'effectue dans un milieu constitué d'un mélange de milieu Dulbecco modifié Eagle et de milieu HAM F12 en une proportion de 3 pour 1, contenant 10 % de sérum de veau foetal, 10 ng/ml de facteur de croissance épidermique (EGF), 400 ng/ml d'hydrocortisone, 10⁻⁶ M Isoprotérénol 5 µg/ml de transferrine, 2 x 10⁻⁹ M de triiodothyronine, 1,8 x 10⁻⁴ M d'adénine et 5 µg/ml d'insuline (Rheinwald et Green, Cell, 1975, 6, 331-334).
La culture est ainsi maintenue immergée pendant 6 jours. La culture est alors placée à l'interface air/liquide, ledit liquide étant alors constitué du même milieu que précédemment duquel l'isoprotérénol, la transferrine, la triiodothyronine et l'adénine ont été retirés.
Parallèlement, des cellules CD34⁺ sont isolés à partir de sang de cordon ombilical et cultivées selon la méthode décrite dans Caux et Coll., (Nature, Vol. 360, 19 novembre 1992, pp. 258-260), pendant 6 jours en présence de facteur de stimulation de colonies (Granulocyte/Macrophage - Colony Stimulating Factor ou GM-CSF) à la concentration de 200 ng/ml et de facteur de nécrose tumorale (Tumor Necrosis Factor ou TNF-α) à la concentration de 2,5 ng/ml. 2 jours après passage du mélange kératinocytes/mélanocytes à l'interface air/liquide, on dépose sur l'épiderme en reconstruction 5x10⁵ cellules CD34⁺, telles que préalablement préparées. La culture est ensuite entretenue jusqu'à ' obtention d'un équivalent d'épiderme histologiquement satisfaisant c'est à dire un équivalent d'épiderme présentant les couches cellulaires classiques, à savoir les couches basale, suprabasale, granuleuse et cornée.

### Préparation d'un équivalent d'épiderme contenant des kératinocytes et des cellules de Langerhans, sans préinduction des précurseurs de cellules de Langerhans :

On constitue un mélange de kératinocytes humains normaux préalablement isolés selon la méthode décrite dans Régnier et coll., (Frontier of Matrix Biology, Vol.9, 4-35, Karger, Basel 1981) et de cellules CD34⁺ isolées à partir de sang de cordon ombilical selon la méthode décrite dans Caux et Coll., (Nature, Vol. 360, 19 novembre 1992, pp. 258-260), dans une proportion de 1 pour 1. Ce mélange est déposé sur un derme désépidermisé à raison de 5x10⁵ cellules de chaque type par cm², selon la méthode décrite dans Prunieras et coll., (Ann. Chir. Plast., 1979, 24, n°4, 357-362). La co-culture s'effectue dans un milieu constitué d'un mélange de milieu Dulbecco modifié Eagle et de milieu HAM F12 en un rapport volumique de 3 pour 1, contenant 10 % de sérum de veau foetal, 10 ng/ml de facteur de croissance épidermique (EGF), 400 ng/ml d'hydrocortisone, 10⁻⁶ M Isoprotérénol 5 µg/ml de transferrine, 2 x 10⁻⁹ M de triiodothyronine, 1,8 x 10⁻⁴ M d'adénine et 5 µg/ml d'insuline (Rheinwald et Green, Cell, 1975, 6, 331-334). La culture est ainsi maintenue immergée pendant 6 jours. La culture est alors placée à l'interface air/liquide, ledit liquide étant alors constitué du même milieu que précédemment duquel l'isoprotérénol, la transferrine, la triiodothyronine et l'adénine ont été retirés.

La co-culture est ensuite entretenue jusqu'à obtention d'un équivalent d'épiderme histologiquement satisfaisant, c'est à dire un équivalent d'épiderme présentant les couches cellulaires classiques, à savoir les couches basale, suprabasale, granuleuse et cornée.

## Revendications

1. Equivalent de peau, **caractérisé par le fait qu'**il comprend un équivalent d'épiderme sur un support, ledit équivalent d'épiderme comprenant au moins des kératinocytes et au moins des précurseurs de cellules de Langerhans induits ou non, les précurseurs de cellules de Langerhans étant des cellules hématopoïétiques CD34⁺.

2. Equivalent de peau selon la revendication 1, **caractérisé par le fait que** le support est choisi parmi les lattices mixtes collagène/fibroblastes, le derme préalablement désépidermisé, les membranes artificielles comme par exemple les filtres de marque Millipores, les substituts sous-cutanés à base de collagène, le plastique ou tout autre support compatible avec la viabilité cellulaire.

3. Equivalent de peau selon la revendication 2, **caractérisé par le fait que** le support est constitué par du derme préalablement désépidermisé.

4. Equivalent de peau selon l'une quelconque des revendications 1 à 3, **caractérisé par le fait qu'**il comprend en outre des mélanocytes.

5. Equivalent de peau selon l'une quelconque des revendications 1 à 4, **caractérisé par le fait que** les précurseurs de cellules de Langerhans induits ou non ont une localisation suprabasale dans l'équivalent d'épiderme.

6. Procédé de préparation d'un équivalent de peau, **caractérisé par le fait que** l'on co-cultive sur un support des kératinocytes et au moins des précurseurs de cellules de Langerhans induits ou non, les précurseurs de cellules de Langerhans étant des cellules hématopoïétiques CD34⁺.

7. Procédé selon la revendication 6, **caractérisé par le fait que** le rapport entre le nombre de kératinocytes et les précurseurs de cellules de Langerhans induits ou non, co-cultivés sur le support est compris entre les rapport 95/5 et 25/75 en pourcentage du nombre total de cellules dans la co-culture et préférentiellement entre 75/25 et 35/65 et encore plus préférentiellement ce rapport est de 50/50.

8. Procédé selon l'une quelconque des revendications 6 ou 7, **caractérisé par le fait que** les kératinocytes sont obtenus par la culture à partir d'épiderme dissocié provenant de prélèvement de peau normale ou pathologique ou par la culture de kératinocytes issus de la gaine de follicule pileux normaux ou pathologiques.

9. Procédé selon la revendication précédente, **caractérisé par le fait que** les kératinocytes sont obtenus à partir de prélèvement de peau normale ou pathologique.

10. Procédé selon l'une quelconque des revendications 6 à 9, **caractérisé par le fait que** les précurseurs des cellules de Langerhans sont purifiés à partir de moelle osseuse, de sang périphérique ou de sang de cordon ombilical.

11. Procédé selon la revendication précédente, **caractérisé par le fait que** les précurseurs des cellules de Langerhans sont purifiés à partir de sang de cordon ombilical.

12. Procédé selon l'une quelconque des revendications 6 à 11, **caractérisé par le fait que** la différenciation des précurseurs de cellules de Langerhans est induite avant ou après leur mise en co-culture.

13. Procédé selon la revendication précédente, **caractérisé par le fait que** la différenciation des précurseurs de cellules de Langerhans est induite après leur mise en co-culture.

14. Procédé selon l'une quelconque des revendications 12 ou 13, **caractérisé par le fait que** la différenciation des précurseurs de cellules de Langerhans est induite selon une méthode choisie parmi la culture en présence de kératinocytes, la culture dans un milieu dans lequel ont été préalablement cultivés des kératinocytes, la culture en présence d'une cytokine choisie parmi le facteur de stimulation de colonies, le facteur de nécrose tumorale, le facteur de cellules souches l'interleukine 3 ou l'interleukine 4.

15. Procédé selon la revendication précédente, **caractérisé par le fait que** la différenciation est induite par la combinaison d'au moins deux des méthodes choisies parmi la culture en présence de kératinocytes, la culture dans un milieu dans lequel ont été préalablement cultivés des kératinocytes, la culture en présence d'une cytokine choisie parmi le facteur de stimulation de colonies, le facteur de nécrose tumorale, le facteur de cellules souches l'interleukine 3 ou l'interleukine 4.

16. Procédé selon la revendication 15, **caractérisé par le fait que** la différenciation est induite par la culture en présence de kératinocytes et en présence d'au moins une cytokine ou par la culture en présence d'un mélange de cytokines, comme par exemple la combinaison du GM-CSF et du TNF-α.

17. Procédé selon la revendication précédente, **caractérisé par le fait que** la différenciation est induite par un mélange de GM-CSF et du TNF-α.

18. Procédé selon l'une quelconque des revendications 6 à 17, **caractérisé par le fait que** les cytokines sont présentes à des concentrations comprises entre 1 ng/ml et 400 ng/ml de préférence entre 2,5 ng/ml et 300 ng/ml.

19. Procédé selon l'une quelconque des revendications 6 à 18, **caractérisé par le fait que** la concentration en GM-CSF est comprise entre 100 ng/ml et 400 ng/ml et de préférence entre 200 ng/ml et 300 ng/ml.

20. Procédé selon l'une quelconque des revendications 6 à 19, **caractérisé par le fait que** la concentration en TNF-α est comprise entre 1ng/ml et 7,5 ng/ml et de préférence entre 2,5 ng/ml et 5 ng/ml.

21. Procédé selon la revendication 17, **caractérisé par le fait que** la proportion entre cytokines dans le cas du mélange GM-CSF/TNF-α est comprise entre les rapports pondéraux 400/1 et 13/1 et de préférence entre 120/1 et 40/1.

22. Procédé selon l'une quelconque des revendications 6 à 21, **caractérisé par le fait que** les précurseurs des cellules de Langerhans induits ou non sont mis en co-culture avec les kératinocytes en un temps permettant leur localisation dans la partie suprabasale de l'équivalent d'épiderme.

23. Procédé selon la revendication précédente, **caractérisé par le fait que** les précurseurs des cellules de Langerhans induits ou non sont mis en culture à un temps choisi entre le moment où la couche suprabasale de l'équivalent d'épiderme commence à se former et le moment où les kératinocytes différenciés commencent à former la première couche du stratum corneum.

24. Procédé selon la revendication 23, **caractérisé par le fait que** les précurseurs des cellules de Langerhans induits ou non sont mis en co-culture à un temps choisi entre le moment où la couche suprabasale de l'équivalent d'épiderme commence à se former et le deuxième jour de culture après passage à l'interface air/liquide.

25. Procédé selon l'une quelconque des revendications 6 à 24, **caractérisé par le fait que** l'on co-cultive en outre des mélanocytes.

26. Equivalent d'épiderme, **caractérisé par le fait qu'**il comprend au moins des kératinocytes et des précurseurs des cellules de Langerhans induits ou non, les précurseurs de cellules de Langerhans étant des cellules hématopoïétiques CD34⁺.

27. Equivalent d'épiderme selon la revendication 26, **caractérisé par le fait qu'**il comprend en outre des mélanocytes.

28. Equivalent d'épiderme selon l'une quelconque des revendications 26 ou 27, **caractérisé par le fait que** les précurseurs des cellules de Langerhans induits ou non ont une localisation suprabasale.

## Claims

1. Skin equivalent, **characterized in that** it comprises an epidermis equivalent on a support, said epidermis equivalent comprising at least keratinocytes and at least induced or non-induced Langerhans cell precursors, the Langerhans cell precursors being CD34⁺ haematopoietic cells.

2. Skin equivalent according to Claim 1, **characterized in that** the support is chosen from collagen/fibroblast mixed lattices, previously de-epidermilized dermis, artificial membranes such as, for example, Millipore filters, collagen-based subcutaneous substitutes, plastic or any other support compatible with cell viability.

3. Skin equivalent according to Claim 2, **characterized in that** the support consists of previously de-epidermilized dermis.

4. Skin equivalent according to any one of Claims 1 to 3, **characterized in that** it also comprises melanocytes.

5. Skin equivalent according to any one of Claims 1 to 4, **characterized in that** the induced or non-induced Langerhans cell precursors have a suprabasal location in the epidermis equivalent.

6. Method for preparing a skin equivalent, **characterized in that** keratinocytes and at least induced or non-induced Langerhans cell precursors are co-cultured on a support, the Langerhans cell precursors being CD34⁺ haematopoietic cells.

7. Method according to Claim 6, **characterized in that** the ratio of the number of keratinocytes to the induced or non-induced Langerhans cell precursors, co-cultured on the support, is between the ratios 95/5 and 25/75 as a percentage of the total number of cells in the co-culture, and preferably between 75/25 and 35/65, and even more preferably this ratio is 50/50.

8. Method according to either one of Claims 6 and 7, **characterized in that** the keratinocytes are obtained by culturing from dissociated epidermis originating from a normal or pathological skin sample or by culturing normal or pathological hair follicle sheath-derived keratinocytes.

9. Method according to the preceding claim, **characterized in that** the keratinocytes are obtained from a normal or pathological skin sample.

10. Method according to any one of Claims 6 to 9, **characterized in that** the Langerhans cell precursors are purified from bone marrow, from peripheral blood or from umbilical cord blood.

11. Method according to the preceding claim, **characterized in that** the Langerhans cell precursors are purified from umbilical cord blood.

12. Method according to any one of Claims 6 to 11, **characterized in that** the differentiation of the Langerhans cell precursors is induced before or after they are placed in co-culture.

13. Method according to the preceding claim, **characterized in that** the differentiation of the Langerhans cell precursors is induced after they are placed in co-culture.

14. Method according to either one of Claims 12 and 13, **characterized in that** the differentiation of the Langerhans cell precursors is induced according to a method chosen from culturing in the presence of keratinocytes, culturing in a medium in which keratinocytes have been cultured beforehand, and culturing in the presence of a cytokine chosen from colony-stimulating factor, tumour necrosis factor, stem cell factor, interleukin 3 and interleukin 4.

15. Method according to the preceding claim, **characterized in that** the differentiation is induced by the combination of at least two of the methods chosen from culturing in the presence of keratinocytes, culturing in a medium in which keratinocytes have been cultured beforehand, and culturing in the presence of a cytokine chosen from colony-stimulating factor, tumour necrosis factor, stem cell factor, interleukin 3 and interleukin 4.

16. Method according to Claim 15, **characterized in that** the differentiation is induced by culturing in the presence of keratinocytes and in the presence of at least one cytokine, or by culturing in the presence of a mixture of cytokines, such as, for example, the combination of GM-CSF and TNF-α.

17. Method according to the preceding claim, **characterized in that** the differentiation is induced by a mixture of GM-CSF and TNF-α.

18. Method according to any one of Claims 6 to 17, **characterized in that** the cytokines are present at concentrations of between 1 ng/ml and 400 ng/ml, preferably between 2.5 ng/ml and 300 ng/ml.

19. Method according to any one of Claims 6 to 18, **characterized in that** the concentration of GM-CSF is between 100 ng/ml and 400 ng/ml, and preferably between 200 ng/ml and 300 ng/ml.

20. Method according to any one of Claims 6 to 19, **characterized in that** the concentration of TNF-α is between 1 ng/ml and 7.5 ng/ml, and preferably between 2.5 ng/ml and 5 ng/ml.

21. Method according to Claim 17, **characterized in that** the proportion between cytokines in the case of the GM-CSF/TNF-α mixture is between the weight ratios 400/1 and 13/1, and preferably between 120/1 and 40/1.

22. Method according to any one of Claims 6 to 21, **characterized in that** the induced or non-induced Langerhans cell precursors are placed in co-culture with the keratinocytes for a period of time which allows them to be located in the suprabasal part of the epidermis equivalent.

23. Method according to the preceding claim, **characterized in that** the induced or non-induced Langerhans cell precursors are placed in culture at a time selected between the moment when the suprabasal layer of the epidermis equivalent begins to form and the moment when the differentiated keratinocytes begin to form the first layer of the stratum corneum.

24. Method according to Claim 23, **characterized in that** the induced or non-induced Langerhans cell precursors are placed in co-culture at a time selected between the moment when the suprabasal layer of the epidermis equivalent begins to form and the second day of culturing after moving to the air/liquid interface.

25. Method according to any one of Claims 6 to 24, **characterized in that** melanocytes are also co-cultured.

26. Epidermis equivalent, **characterized in that** it comprises at least keratinocytes and induced or non-induced Langerhans cell precursors, the Langerhans cell precursors being CD34⁺ haematopoietic cells.

27. Epidermis equivalent according to Claim 26, **characterized in that** it also comprises melanocytes.

28. Epidermis equivalent according to either one of Claims 26 and 27, **characterized in that** the induced or non-induced Langerhans cell precursors have a suprabasal location.

## Patentansprüche

1. Hautäquivalent, **dadurch gekennzeichnet, dass** es ein Epidermisäquivalent auf einem Träger enthält, wobei das Epidermisäquivalent zumindest Keratinocyten und zumindest induzierte oder nicht induzierte Vorläufer von Langerhans-Zellen enthält und wobei die Vorläufer von Langerhans-Zellen hämatopoetische CD34⁺-Zellen sind.

2. Hautäquivalent nach Anspruch 1, **dadurch gekennzeichnet, dass** der Träger unter den gemischten Kollagen/Fibroblasten-Gittern, zuvor von der Epidermis befreiter Dermis, künstlichen Membranen, wie beispielsweise Filtern der Marke Millipor, Unterhautsubstituten auf Kollagenbasis, Kunststoff oder beliebigen weitern, mit der Lebensfähigkeit der Zellen verträglichen Trägern ausgewählt ist.

3. Hautäquivalent nach Anspruch 2, **dadurch gekennzeichnet, dass** der Träger aus einer zuvor von der Epidermis befreiten Dermis besteht.

4. Hautäquivalent nach einem der Ansprüche 1 bis 3, **dadurch gekennzeichnet, dass** es ferner Melanocyten enthält.

5. Hautäquivalent nach einem der Ansprüche 1 bis 4, **dadurch gekennzeichnet, dass** die induzierten oder nicht induzierten Vorläufer von Langerhans-Zellen in dem Epidermisäquivalent suprabasal lokalisiert sind.

6. Verfahren zur Herstellung eines Hautäquivalents, **dadurch gekennzeichnet, dass** auf einem Träger Keratinocyten und zumindest induzierte oder nicht induzierte Vorläufer von Langerhans-Zellen kokultiviert werden, wobei die Vorläufer von Langerhans-Zellen hämatopoetische CD34⁺-Zellen sind.

7. Verfahren nach Anspruch 6, **dadurch gekennzeichnet, dass** das Verhältnis der Anzahl der Keratinocyten und der induzierten oder nicht induzierten Vorläufer von Langerhans-Zellen, die auf dem Träger kokultiviert werden, im Bereich von 95/5 bis 25/75, ausgedrückt als prozentualer Anteil der Gesamtzahl der Zellen in der Kokultur, und vorzugsweise im Bereich von 75/25 bis 35/65 liegt, wobei das Verhältnis noch bevorzugter 50/50 beträgt.

8. Verfahren nach einem der Ansprüche 6 oder 7, **dadurch gekennzeichnet, dass** die Keratinocyten durch Kultur ausgehend von dissoziierter Epidermis, die aus Biopsien von normaler oder pathologischer Haut stammt, oder durch Kultur von Keratinocyten aus der Hülle von normalen oder pathologischen Haarfollikeln erhalten werden.

9. Verfahren nach dem vorhergehenden Anspruch, **dadurch gekennzeichnet, dass** die Keratinocyten aus Entnahmen normaler oder pathologischer Haut erhalten werden.

10. Verfahren nach einem der Ansprüche 6 bis 9, **dadurch gekennzeichnet, dass** die Vorläufer von Langerhans-Zellen ausgehend von Knochenmark, peripherem Blut oder Nabelschnurblut in gereinigter Form erhalten werden.

11. Verfahren nach dem vorhergehenden Anspruch, **dadurch gekennzeichnet, dass** die gereinigten Vorläufer von Langerhans-Zellen aus Nabelschnurblut erhalten werden.

12. Verfahren nach einem der Ansprüche 6 bis 11, **dadurch gekennzeichnet, dass** die Differenzierung der Vorläufer von Langerhans-Zellen induziert wird, bevor sie kokultiviert werden oder nachdem die Kokultur begonnen wurde.

13. Verfahren nach dem vorhergehenden Anspruch, **dadurch gekennzeichnet, dass** die Differenzierung der Vorläufer von Langerhans-Zellen induziert wird, nachdem ihre Kokultur begonnen wurde.

14. Verfahren nach einem der Ansprüche 12 oder 13, **dadurch gekennzeichnet, dass** die Differenzierung der Vorläufer von Langerhans-Zellen nach einem Verfahren induziert wird, das unter der Kultur in Gegenwart von Keratinocyten, der Kultur in einem Medium, in dem zuvor Keratinocyten kultiviert wurden, und der Kultur in Gegenwart eines Cytokins ausgewählt ist, welches unter dem Kolonie-Stimulierenden-Faktor, dem Tumomekrosefaktor, dem Stammstellenfaktor, Interleukin 3 oder Interleukin 4 ausgewählt ist.

15. Verfahren nach dem vorhergehenden Anspruch, **dadurch gekennzeichnet, dass** die Differenzierung durch die Kombination von mindestens zwei Verfahren induziert wird, die unter der Kultur in Gegenwart von Keratinocyten, der Kultur in einem Medium, in dem zuvor Keratinocyten kultiviert wurden, und der Kultur in Gegenwart eines Cytokins ausgewählt sind, welches unter dem Kolonie-Stimulierenden-Faktor, dem Tumornekrosefaktor, dem Stammzellenfaktor, Interleukin 3 oder Interleukin 4 ausgewählt ist.

16. Verfahren nach Anspruch 15, **dadurch gekennzeichnet, dass** die Differenzierung durch die Kultur in Gegenwart von Keratinocyten und in Gegenwart mindestens eines Cytokins oder durch die Kultur in Gegenwart eines Gemisches von Cytokinen, wie beispielsweise der Kombination von GM-CSF und TNF-α, induziert wird.

17. Verfahren nach dem vorhergehenden Anspruch, **dadurch gekennzeichnet, dass** die Differenzierung durch ein Gemisch von GM-CSF und TNF-α induziert wird.

18. Verfahren nach einem der Ansprüche 6 bis 17, **dadurch gekennzeichnet, dass** die Cytokine in Konzentrationen von 1 bis 400 ng/ml und vorzugsweise 2,5 bis 300 ng/ml vorliegen.

19. Verfahren nach einem der Ansprüche 6 bis 18, **dadurch gekennzeichnet, dass** die Konzentration des GM-CSF im Bereich von 100 bis 400 ng/ml und vorzugsweise 200 bis 300 ng/ml liegt.

20. Verfahren nach einem der Ansprüche 6 bis 19, **dadurch gekennzeichnet, dass** die Konzentration des TNF-α im Bereich von 1 bis 7,5 ng/ml und vorzugsweise 2,5 bis 5 ng/ml liegt.

21. Verfahren nach Anspruch 17, **dadurch gekennzeichnet, dass** im Falle des Gemisches GM-CSF/TNF-α das Verhältnis der Cytokine im Bereich der Gewichtsverhältnisse 400/1 bis 13/ 1 und vorzugsweise 120/1 bis 40/1 liegt.

22. Verfahren nach einem der Ansprüche 6 bis 21, **dadurch gekennzeichnet, dass** die induzierten oder nicht induzierten Vorläufer von Langerhans-Zellen mit den Keratinocyten zu einem Zeitpunkt kokultiviert werden, der ihre Lokalisierung in dem suprabasalen Bereich des Epidermisäquivalents ermöglicht.

23. Verfahren nach dem vorhergehenden Anspruch, **dadurch gekennzeichnet, dass** die induzierten oder nicht induzierten Vorläufer von Langerhans-Zellen zu einem Zeitpunkt kultiviert werden, der im Bereich des Zeitpunkts, bei dem sich die suprabasale Schicht des Epidermisäquivalents zu bilden beginnt, und des Zeitpunkts, bei dem die differenzierten Keratinocyten die erste Schicht des *Stratum corneum* zu bilden beginnen, ausgewählt ist.

24. Verfahren nach Anspruch 23, **dadurch gekennzeichnet, dass** die induzierten oder nicht induzierten Vorläufer von Langerhans-Zellen zu einem Zeitpunkt kokultiviert werden, der im Bereich des Zeitpunkts, bei dem sich die suprabasale Schicht des Epidermisäquivalents zu bilden beginnt, und dem zweiten Tag der Kultur nach dem Überführen an die Grenzfläche Luft/ Flüssigkeit ausgewählt ist.

25. Verfahren nach einem der Ansprüche 6 bis 24, **dadurch gekennzeichnet, dass** außerdem Melanocyten kokultiviert werden.

26. Epidermisäquivalent, **dadurch gekennzeichnet, dass** es zumindest Keratinocyten und induzierte oder nicht induzierte Vorläufer von Langerhans-Zellen enthält, wobei die Vorläufer von Langerhans-Zellen hämatopoetische CD34⁺-Zellen sind.

27. Epidermisäquivalent nach Anspruch 26, **dadurch gekennzeichnet, dass** es außerdem Melanocyten enthält.

28. Epidermisäquivalent nach einem der Ansprüche 26 oder 27, **dadurch gekennzeichnet, dass** die induzierten oder nicht induzierten Vorläufer von Langerhans-Zellen suprabasal lokalisiert sind.
